Europäisches Patentamt

European Patent Office

Office européen des brevets

(19)

(11) **EP 1 481 662 A1**

(12) ## DEMANDE DE BREVET EUROPEEN

(43) Date de publication:
**01.12.2004 Bulletin 2004/49**

(51) Int Cl.⁷: **A61K 7/043**

(21) Numéro de dépôt: **04291227.9**

(22) Date de dépôt: **13.05.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **26.05.2003 FR 0306357**

(71) Demandeur: **L'OREAL**
**75008 Paris (FR)**

(72) Inventeurs:
• **Ramin, Roland**
  **75014 Paris (FR)**
• **Cavazzuti, Roberto**
  **75015 Paris (FR)**

(74) Mandataire: **Boulard, Denis**
  **L'OREAL - D.I.P.I.**
  **25-29 Quai Aulagnier**
  **92600 Asnières (FR)**

(54) **Kit de maquillage ou de soin des ongles**

(57) La présente invention a pour objet un kit de maquillage ou de soin des ongles comprenant :

i) une première composition comprenant, dans un premier milieu cosmétiquement acceptable un premier polymère filmogène, la première composition étant apte à former un film ayant une température de transition vitreuse Tg allant de 25 à 60°C,

ii) une seconde composition comprenant un second milieu cosmétiquement acceptable, la seconde composition ayant un extrait sec supérieur ou égal à 15%.

EP 1 481 662 A1

## Description

La présente invention a pour objet un kit de maquillage ou de soin des ongles associant deux compositions et permettant l'obtention d'un film sur l'ongle ayant une tenue améliorée. L'invention a également se rapporte également au procédé de maquillage ou de soin des ongles correspondant.

Il est connu de l'art antérieur des compositions de vernis à ongles visant à augmenter la tenue du maquillage des ongles. Ainsi, la demande de brevet EP-A-1 082 952 décrit des compositions de maquillage, notamment des ongles, contenant des particules de verre recouvertes d'une couche métallique permettant l'obtention d'un maquillage présentant un aspect métallique éclatant et résistant à l'usure. On connaît également le brevet EP 0 813 858 qui divulgue l'utilisation d'acide silicique sous forme colloïdale dans une composition pour les ongles pour améliorer la tenue et la résistance du film de vernis à ongles. Enfin, le document WO-A-00/27347 a pour objet des composition pour les ongles comprenant des microsphères permettant d'améliorer la tenue et l'adhésion du film de composition déposé sur les ongles.

Les inventeurs ont découvert qu'il était possible d'obtenir des films de vernis à ongles présentant une tenue globale (en particulier une résistance à l'usure, aux frottements et aux chocs) améliorée par rapport à ceux obtenus dans l'art antérieur en utilisant un kit de maquillage associant deux compositions.

De façon plus précise, l'invention a pour objet un kit de maquillage ou de soin des ongles comprenant :

i) une première composition comprenant, dans un premier milieu cosmétiquement acceptable un premier polymère filmogène, la première composition étant apte à former un film ayant une température de transition vitreuse Tg allant de 25 à 60°C,

ii) une seconde composition comprenant un second milieu cosmétiquement acceptable, la seconde composition ayant un extrait sec supérieur ou égal à 15%.

L'invention a également pour objet un procédé cosmétique de maquillage ou soin non thérapeutique des ongles consistant à appliquer sur les ongles une première couche d'une première composition comprenant un premier milieu cosmétiquement acceptable et un premier polymère filmogène, la première composition étant apte à former un film ayant une température de transition vitreuse Tg allant de 25 à 60°C, puis à appliquer sur tout ou partie de la première couche et avant séchage de la première couche, une seconde couche d'une seconde composition comprenant un second milieu cosmétiquement acceptable, la seconde composition ayant un extrait sec supérieur ou égal à 15%.

L'invention a aussi pour objet un procédé cosmétique de maquillage ou soin non thérapeutique des ongles consistant à appliquer sur les ongles une première couche d'une première composition comprenant un premier milieu cosmétiquement acceptable et un premier polymère filmogène, la première composition étant apte à former un film ayant une température de transition vitreuse Tg allant de 25 à 60°C, puis, après séchage total ou partiel de la première couche, à appliquer sur tout ou partie de la première couche une seconde couche d'une seconde composition comprenant un second milieu cosmétiquement acceptable, la seconde composition ayant un extrait sec supérieur ou égal à 15%.

Par séchage partiel de la première couche, on entend un séchage superficiel mais néanmoins suffisant de la première couche afin que la seconde couche puisse être appliquée sans endommager ladite première couche.

L'invention a encore pour objet l'utilisation d'un kit tel que décrit ci-dessus, pour obtenir un film déposé sur l'ongle, présentant des propriétés de tenue et/ou une résistance à l'usure améliorée.

Par "milieu cosmétiquement acceptable", on entend au sens de l'invention un milieu compatible avec le maquillage des ongles.

### Première composition

La première composition est apte à former un film ayant une température de transition vitreuse Tg allant de 25 à 60°C, de préférence de 30 à 45°C et forme ainsi sur l'ongle un film dit "mou" qui présente une bonne adhésion sur l'ongle et une bonne résistance au chocs du fait de sa capacité à les absorber. Si la Tg du film de première composition est en dessous de 25°C, le film formé est trop mou, trop collant, il ne présente pas une bonne filmification et ne permet pas l'application homogène de la seconde composition. Si la Tg du film de composition va au-delà de 60°C, le film de composition est trop dur, et donc fragile et cassant et ne permet pas un résultat final satisfaisant, notamment en matière de tenue et/ou de résistance à l'usure.

### Méthode de mesure de la température de transition vitreuse Tg

La mesure de la température de transition vitreuse Tg du film de composition est effectuée par DMTA (Dynamical and Mechanical Temperature Analysis ou Analyse dynamique et mécanique en température) avec un appareil DMTA de Polymer TA Instruments (modèle DMA2980) sur un échantillon de film de composition.

L'échantillon est préparé par coulage de la composition dans une matrice téflonnée puis séchage sur plaque thermostatée à 30 °C pendant 24 heures, sous des conditions d'humidité ambiante (typiquement 50% HR ± 15%). On obtient alors un film dans lequel on découpe (par exemple à l'emporte-pièce) les éprouvettes. Celles-ci ont typiquement une épaisseur d'environ 200 µm, une largeur d'environ 10 mm et une longueur utile d'environ 7 mm, après 24h de séchage. L'échantillon est sollicité en traction et en petites déformations à la fréquence de 1 Hz (on lui impose par exemple un déplacement sinusoïdal de ± 8 µm) lors d'un balayage en température à 3°C/min. Cette sollicitation de traction est effectuée sur l'échantillon à des températures variant par exemple de -10°C à + 80 °C.

On détermine le module complexe $E^* = E' + iE''$ du polymère testé en fonction de la température, E' étant le module de conservation et E'' le module dit de perte.

De ces mesures, on déduit également le pouvoir amortissant : $tg\delta = E''/E'$.

Puis on trace la courbe des valeurs de $tg\delta$ en fonction de la température. La température de transition vitreuse Tg du polymère correspond à la température pour laquelle on obtient une valeur maximale (correspondant à un pic) de $tg\delta$. Lorsque la courbe présente au moins 2 pics (dans ce cas, le polymère présente au moins 2 relaxations), on prend comme valeur de Tg du polymère testé la température pour laquelle la courbe présente le pic de plus forte amplitude (c'est à dire correspondant à la plus grande valeur de $tg\delta$ ; on ne considère dans ce cas que la Tg « majoritaire » comme valeur de Tg du polymère testé).

Ce film mou de première composition peut également être caractérisé par sa dureté Persoz. La dureté du film est mesurée pour une couche de 300 µm d'épaisseur (avant séchage), déposée sur une plaque de verre. La plaque de verre est séchée sur un support et maintenue à 30 °C pendant 23 heures dans une atmosphère ambiante, puis pendant 1 heure, à 70 % d'humidité relative. La dureté du film obtenu est mesurée selon la norme

ASTM D-43-66, ou la norme NF-T 30-016 (décembre 1991), à l'aide d'un pendule de Persoz.

Avantageusement, la première composition est apte à former un film ayant une dureté de 30 à 60 secondes.

En particulier, la première composition selon l'invention est apte à former un film caractérisé par un comportement viscoélastique particulier.

De façon générale, un matériau est dit viscoélastique quand, sous l'effet du cisaillement, il possède à la fois les caractéristiques d'un matériau purement élastique, c'est à dire capable de stocker de l'énergie et les caractéristiques d'un matériau purement visqueux, c'est à dire capable de dissiper de l'énergie et dont la réponse aux sollicitations est fonction du temps (réponse non instantanée).

Plus particulièrement la première composition selon l'invention peut être caractérisé par son pouvoir amortissant $tg\delta$, qui représente le rapport entre l'énergie dissipée et l'énergie transmise au sein du matériau.

Avantageusement, la première composition est apte à former un film ayant un pouvoir amortissant tangente delta ($tg\delta$) supérieur ou égal à 0,7 à 25°C et 20 Hz.

Methodes de mesure du pouvoir amortissant $tg\delta$

La mesure du pouvoir amortissant $tg\delta$ est effectuée par DMTA selon la méthode précédemment décrite mais avec les différences suivantes :

- les mesures sont effectués sur un échantillon de film de polymère d'une épaisseur inférieur à 200 ± 50 µm d'épaisseur, d'environ 10 mm de largeur et d'environ 7 mm de longueur, à une température constante de 25°C.
- l'échantillon est sollicité en traction et en petites déformations (on lui impose par exemple un déplacement sinusoïdal de ± 8 µm) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe $E^* = E' + iE''$ du film de composition testé, E' étant le module de conservation et E'' le module dit de perte.

De ces mesures, on déduit le pouvoir amortissant : $tg\delta = E''/E'$.

Selon un mode préféré de réalisation, la première composition du kit selon l'invention comprend des particules solides de forme sphérique.

Par "particules de forme sphérique", on entend des particules de forme sphérique ou sensiblement sphérique (par exemple de forme ovoïdale).

Selon un second aspect, la présente invention a donc pour objet un kit de maquillage ou de soin des ongles comprenant :

i) une première composition comprenant, dans un premier milieu cosmétiquement acceptable un premier polymère filmogène et des particules solides de forme sphérique,

ii) une seconde composition comprenant un second milieu cosmétiquement acceptable, ladite composition ayant un extrait sec supérieur ou égal à 15%.

La présente invention a également pour objet un procédé cosmétique de maquillage ou de soin non thérapeutique des ongles consistant à appliquer sur les ongles une première couche d'une première composition comprenant un premier milieu cosmétiquement acceptable, un premier polymère filmogène et des particules solides de forme sphérique, puis à appliquer sur tout ou partie de la première couche et avant séchage de la première couche, une seconde couche d'une seconde composition comprenant un second milieu cosmétiquement acceptable, la seconde composition ayant un extrait sec supérieur ou égal à 15%.

La présente invention a également pour objet un procédé cosmétique de maquillage ou de soin non thérapeutique des ongles consistant à appliquer sur les ongles une première couche d'une première composition comprenant un premier milieu cosmétiquement acceptable, un premier polymère filmogène et des particules solides de forme sphérique, puis, après séchage total ou partiel de la première couche, à appliquer sur tout ou partie de la première couche une seconde couche d'une seconde composition comprenant un second milieu cosmétiquement acceptable, la seconde composition ayant un extrait sec supérieur ou égal à 15%.

Les particules sphériques utilisées dans les compositions selon l'invention ont de préférence une taille moyenne inférieure ou égale à 50 μm, par exemple allant de 0,5 à 30 μm, de préférence de 5 à 15 μm et notamment d'environ 10 μm.

Par « taille moyenne », on désigne la dimension donnée par la distribution granulométrique statistique à la moitié de la population, dite D50.

Les particules solides de forme sphérique peuvent être en un matériau choisi parmi :

le verre, les céramiques, les alumines, les silices, les silicates et notamment les aluminosilicates et les borosilicates, le mica, le mica synthétique tel que la fluorophlogopite, le carbure de tungstène, la cellulose et ses dérivés, les résines de silicone, les polymères tels que les polyuréthanes, les polyamides, les polymères acryliques, le polytétrafluoroéthylène, le polypropylène, les cires naturelles ou synthétiques, les métaux comme l'acier, le cuivre, l'or, l'argent, le chrome, les oxydes métalliques comme le dioxyde de titane, l'oxyde de fer, l'oxyde de zirconium, l'oxyde de cérium, les oxydes d'alumine, les alliages tels que l'acier, le laiton, les matériaux d'origine végétale comme le bois, d'origine minérale tels que le quartz, les laves ou les alluvions, ou les matériaux issus de sécrétion animale, comme le corail, les nacres et leurs mélanges.

Ces particules solides, molles ou dures, déformables ou non déformables, peuvent être pleines ou creuses, incolores ou colorées.

Les particules creuses peuvent contenir un matériau solide ou non.

Les particules peuvent être enrobées ou recouvertes en totalité ou en partie par au moins une couche de matériau tel que ceux décrits ci-dessus ou d'autres natures.

Elles peuvent être composées de matériaux de natures différentes et non homogènes.

A titre de particules solides de forme sphérique utilisables dans la présente invention, on peut citer notamment :

- les billes de cellulose creuses telles que celles commercialisées sous la référence Covabead CLO par la société DAITO,
- les microsphères de verre commercialisées sous les dénominations Prizmalite par la société Prizmalite,
- les microsphères de silice poreuses telles que celles commercialisées sous les références SA-SB, SI-SB par la société Miyoshi,
- les microbilles de silice commercialisées sous la référence Spheron par la société Catalyst Chemicals,
- les microbilles de résine methylsilsesquioxane commercialisées sous la dénomination TOSPEARL par la société General Electric Toshiba Silicones,
- les micro billes de copolymère ethylène/acide acrylique enrobées de nano titane commercialisées sous la référence EA-209/MT100T par la société Kobo,
- les billes de polyéthylène enrobées de cires synthétiques minérales de polytetrafluoroéthylène commercialisées sous les dénominations Microsilk ou Polyblend par la société Micro Powders,
- les microsphères de borosilicate de calcium et aluminium commercialisées sous la référence Luxsil par la société Potters Industries
- et leurs mélanges.

De préférence, les particules solides de forme sphérique sont des particules de verre, et en particulier des microbilles de verre. Par "verre", on entend toute composition de verre, le verre étant généralement, de façon non limitative, composé d'un mélange d'oxydes choisis parmi les oxydes de silicium, de titane, de barium, d'aluminium, de calcium, de magnésium, de bore, de potassium, de sodium, de zinc et leurs mélanges.

En particulier les particules de verre de forme sphérique utilisées dans la présente invention sont composées des oxydes suivants, dans les teneurs indiquées ci après :

- SiO$_2$ en une teneur allant de 5 à 10%,
- TiO$_2$ en un teneur allant de 25 à 35%,
- BaO en un teneur allant de 45 à 50%,
- CaO en un teneur allant de 1 à 10%,
- B$_2$O$_3$ en une teneur allant de 2 à 10%,
- Al$_2$O$_3$ en une teneur allant de 0,05 à 1% ,
- Na$_2$O en une teneur allant de 0,05 à 1%,
- et d'autres oxydes sous forme de traces.

Les particules solides de forme sphérique peuvent représenter de 0,1 à 30% en poids par rapport au poids total de la composition, de préférence de 0,5 à 10 % et mieux de 1 à 5 % en poids.

La première composition présente avantageusement une viscosité dynamique, à 25 °C et à pression atmosphérique, de l'ordre de 0,15 à 0,5 Pa.s, mesurée à l'aide d'un viscosimètre Brookfield type LV II.

Le choix d'une telle viscosité est favorable à l'organisation physique des particules sphériques au moment de leur application sur l'ongle, et en particulier privilégie l'obtention d'une répartition homogène et continue desdites particules.

Après application de la première composition sur l'ongle, on obtient ainsi, grâce à la présence des particules solides de forme sphérique, un film de composition présentant une irrégularité de surface, ce qui permet, lors de l'application de la seconde composition, un meilleur accrochage de la seconde couche et l'obtention d'un dépôt final sur l'ongle renforcé.

Le premier milieu cosmétiquement acceptable de la première composition peut être un milieu choisi parmi un milieu organique comprenant au moins un solvant organique, un milieu aqueux et leur mélange.

La première composition selon l'invention peut comprendre au moins un milieu solvant organique constituant une phase organique, composé d'au moins un solvant organique. Comme solvant organique volatil ou non à température ambiante, on peut citer :

- les cétones liquides à température ambiante tels que la méthyléthylcétone, la méthylisobutylcétone, la diisobutylcétone, l'isophorone, la cyclohexanone, et l'acétone ;
- les alcools liquides à température ambiante tels que l'éthanol, l'isopropanol, le butanol, le diacétone alcool, le 2-butoxyéthanol, et le cyclohexanol ;
- les glycols liquides à température ambiante tels que l'éthylène glycol, le propylène glycol, le pentylène glycol, et le glycérol ;
- les éthers de propylène glycol liquides à température ambiante tels que le monométhyléther de propylène glycol, l'acétate de monométhyl éther de propylène glycol, et le mono n-butyl éther de dipropylène glycol ;
- les esters à chaînes courtes (ayant de 3 à 8 atomes de carbone au total) tels que l'acétate de méthyle, l'acétate d'éthyle, l'acétate de propyle, l'acétate de n-butyle, l'acétate d'aryle, et l'acétate d'isopentyle ;
- les alcanes liquides à température ambiante tels que le décane, l'heptane, l'octane, le dodécane, le cyclohexane, et l'isododécane ;
- les aldéhydes liquides à température ambiante, tels que le benzaldéhyde et l'acétaldéhyde, et
- leurs mélanges.

Le solvant est en particulier choisi parmi les esters à chaîne courte (ayant de 3 à 8 atomes de carbone au total), les alcools liquides à température ambiante, et leurs mélanges.

La composition à milieu solvant organique peut comprendre, en outre, de l'eau, notamment à une teneur allant de 0,1 à 10% en poids, par rapport au poids total de la composition.

La composition selon l'invention peut aussi comprendre au moins un milieu aqueux, constituant une phase aqueuse, qui peut former la phase continue de la composition.

La phase aqueuse peut être constituée essentiellement d'eau; elle peut également comprendre un mélange d'eau et de solvant(s) organique(s) miscible(s) à l'eau (miscibilité dans l'eau supérieure à 50 % en poids à 25 °C) comme les monoalcools inférieure ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C$_3$-C$_4$, les aldéhydes en C$_2$-C$_4$.

Le milieu solvant organique, le milieu aqueux ou la somme de ces deux phases peut être présent en une teneur totale comprise entre 1 et 90 % en poids, notamment allant de 5 à 60 % en poids, et en particulier allant de 15 à 40 % en poids, par rapport au poids total de ladite première composition.

La première composition comprend en outre au moins un polymère filmogène dit "premier polymère filmogène".

Dans la présente demande, on entend par « polymère filmogène » un polymère apte à former à lui seul ou en présence d'un agent plastifiant éventuel, un film isolable. Le polymère filmogène peut être solubilisé ou dispersé sous forme de particules dans le milieu cosmétiquement acceptable des première et/ou seconde compositions.

Le polymère filmogène peut être choisi parmi les polymères radicalaires, les polycondensats et les polymères d'origine naturelle.

Le polymère filmogène peut être choisi notamment dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

Lorsque la première composition comprend un milieu solvant organique, le polymère filmogène peut être choisi notamment parmi les résines alkydes, acryliques et/ou vinyliques, les polyuréthanes et les polyesters, les celluloses et dérivés cellulosiques, telles que la nitrocellulose, les esters de cellulose, tels que l'acétate de cellulose, l'acétopropionate de cellulose, l'acétobutyrate de cellulose, les résines résultant de la condensation de formaldéhyde avec une arylsulfonamide et leurs mélanges.

Lorsque la première composition comprend un milieu aqueux, alors le polymère filmogène est généralement présent sous la forme de particules en dispersion dans le milieu aqueux formant ainsi un latex ou pseudo latex.

Parmi les polymères filmogènes susceptibles d'être utilisés en milieu aqueux, on peut citer les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange. La dispersion peut également comprendre un polymère associatif de type polyuréthane ou une gomme naturelle, telle que la gomme xanthane.

Comme polymère en dispersion aqueuse, on peut citer les dispersions de polymères acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la Société ZENECA, DOW LATEX 432® , par la Société DOW CHEMICAL. On peut également employer des dispersions aqueuses de polyuréthane, et notamment les polyester-polyuréthanes vendus sous les dénominations « AVALURE UR-405® », «AVALURE UR-410® », « AVALURE UR-425® », «SANCURE 2060® » par la Société GOODRICH et les polyéther-polyuréthanes vendus sous les dénominations « SANCURE 878® » par la Société GOODRICH, « NEOREZ R-970® » par la Société AVECIA.

Le premier polymère filmogène peut être présent en une teneur en matières sèches allant de 1% à 70% en poids, par rapport au poids total de la première composition, et mieux de 10% à 45%.

Pour améliorer les propriétés filmogènes de la première composition de vernis à ongle un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants.

En outre, lorsque la première composition selon l'invention comprend un polymère filmogène sous forme de particules dispersées dans un milieu aqueux, l'agent auxiliaire de filmification peut aussi être choisi parmi les agents de coalescence.

Seconde composition

La seconde composition du kit selon l'invention comprend un second milieu cosmétiquement acceptable et a une teneur en matières sèches ou extrait sec supérieur ou égale à 15%, de préférence supérieur ou égale à 20%, et mieux supérieur ou égale à 25%.

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer (cette dernière méthode étant applicable uniquement dans le cas où la partie volatile de la composition n'est composée que d'eau).

De préférence, la quantité de matière sèche, communément appelée « extrait sec » des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler. Le protocole de mesure est le suivant :

On étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \times (\text{masse sèche} / \text{masse humide})$$

De préférence, la seconde composition présente une viscosité dynamique, à 25 °C et à pression atmosphérique, allant de 0,15 à 1,5 Pa.s, mesurée à l'aide d'un viscosimètre Brookfield type LV II. La seconde composition doit en effet être suffisamment fluide pour permettre l'obtention d'une seconde couche uniforme sur l'ongle, et suffisamment dense pour recouvrir les aspérités de la première couche et permettre la formation d'un film lisse.

Cette seconde composition, destinée à être appliquée sur la première couche de la première composition, permet d'obtenir un film de vernis présentant une bonne résistance à l'usure (en particulier aux frottements).

La résistance du film pouvant être obtenu avec la seconde composition du kit selon l'invention peut être notamment mesurée selon la norme AFNOR NF T30-015, dont le principe est rappelé ci-après.

On applique la composition à tester sous forme d'une couche de 600 µm d'épaisseur (avant séchage) sur un disque, puis on laisse sécher pendant une heure à 30 °C. Le film de vernis déposé sur le disque est ensuite mis pendant une heure en contact avec des disques abrasifs (abrasimètre TABER), le disque ayant une vitesse de rotation d'un tour par seconde. Au bout d'une heure, on pèse le disque et on calcule la perte de masse PM de produit exprimée en pourcentage du poids perdu par rapport au poids initial.

Par conséquent, dans ce test, plus la perte de poids est faible, plus le pourcentage de poids perdu est fiable, et plus la résistance à l'usure de la composition est élevée.

Le film obtenu avec la seconde composition de l'invention présente une résistance à l'usure exprimée en perte de masse mesurée selon la norme AFNOR NF T 30-015 inférieure à 5%.

La seconde composition comprend un second milieu cosmétiquement acceptable qui peut être un milieu solvant organique, aqueux ou mixte, choisi parmi les milieux solvant organique et aqueux décrits plus haut et pouvant être présent dans la seconde composition dans les teneurs indiquées plus haut pour la première composition.

Bien entendu, l'homme du métier veillera à choisir ce second milieu cosmétiquement acceptable, de telle sorte que l'application de la seconde composition sur le film susceptible d'être généré par l'application de la première composition n'affecte pas la qualité dudit film.

La seconde composition du kit selon l'invention comprend de préférence un polymère filmogène dit "second polymère filmogène" choisi parmi les polymères filmogènes décrits plus haut, en une teneur en matières sèches allant de 1% à 70% en poids, par rapport au poids total de la seconde composition.

De préférence, la seconde composition comprend un second polymère filmogène en une teneur en matière sèches supérieure ou égale à 5%, de préférence supérieure à 10%, mieux supérieure à 15%, et encore mieux supérieure à 20%.

La seconde composition du kit de l'invention peut également comprendre au moins un agent auxiliaire de filmification, tels que ceux décrits pour la première composition.

Selon un mode de réalisation, la seconde composition est apte à former un film ayant une brillance supérieure à 70, la brillance du film étant mesurée à l'aide d'un brillancemètre, de manière conventionnelle, par la méthode suivante.

Sur une carte de contraste de marque LENETA et de référence FORM 1A PENOPAC, on étale une couche de 300 µm d'épaisseur de la composition dont on cherche à évaluer la brillance moyenne, à l'aide d'un étaleur automatique. La couche recouvre au moins le fond blanc de la carte. On procède de suite (sur film humide) à la mesure de la brillance à 20° sur le fond blanc à l'aide d'un brillancemètre de marque BYK GARDNER et de référence microTRI-GLOSS.

Selon un autre mode de réalisation, la seconde composition comprend des charges dites matifiantes de manière à obtenir un film plus ou moins mat. Ces charges peuvent être choisies parmi les poudres de nylon ou de soie, les silices précipitées, le talc, le kaolin, le mica, les plaquettes de silice et leurs mélanges. Leur quantité et leur nature est ajustée selon la matité désirée pour le film de seconde composition.

Additifs

La première et/ou la seconde composition peut comprendre, en outre, au moins un agent plastifiant. En particulier, on peut citer, seuls ou en mélange, les plastifiants usuels, tels que :

- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;

- les esters de glycol;
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, des tartrates, des phosphates, des sébaçates ;
- des dérivés oxyéthylénés, tels que les huiles oxyéthylénées, notamment les huiles végétales, telles que l'huile de ricin ;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut, par exemple, aller de 0,1 % à 15 % en poids, par rapport au poids total de chacune des première ou seconde composition, et de préférence de 0,5 % à 10 % en poids.

Chacune des première et/ou seconde composition peut comprendre en outre une matière colorante qui peut être choisie parmi les composés pulvérulents et/ou les colorants solubles dans le milieu de la composition. La matière colorante peut être présente en une teneur allant de 0,01% à 25% en poids et de préférence de 2 à 7% en poids par rapport au poids total de chacune des première ou seconde composition.

Les composés pulvérulents peuvent être choisis parmi les pigments et/ou les nacres et/ou les paillettes, habituellement utilisés dans les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium, le cuivre ou le bronze. Parmi les pigments organiques, on peut citer le noir de carbone, les pigments de type D & C, et les laques à base de carmin de cochenille, de baryum, strontium, calcium, aluminium, la guanine.

On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines et recouvert ou non de substances métalliques comme l'aluminium, l'or , le cuivre, le bronze, ou d'oxydes métalliques comme le dioxyde de titane, l'oxyde de fer, l'oxyde de chrome, de pigments minéraux ou organiques et leurs mélanges.

Les pigments nacrés peuvent être choisis parmi les pigments nacrés blancs, tels que le mica recouvert de titane, ou d'oxychlorure de bismuth, les pigments nacrés colorés, tels que le mica titane avec des oxydes de fer, le mica titane avec notamment du bleu ferrique ou de l'oxyde de chrome, le mica titane avec un pigment organique du type précité ainsi que les fluorophlogopite avec des oxydes de fer.

On peut aussi utiliser des pigments à propriétés goniochromatiques, notamment à cristaux liquides ou multicouches.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.

La matière colorante peut également être choisie parmi les azurants optiques.

Chacune des première et/ou seconde compositions peut comprendre, en outre, tout autre ingrédient, additif, utilisé couramment dans les compositions cosmétiques et connu de l'homme du métier comme étant susceptible d'être incorporé dans une composition de vernis à ongles.

De tels ingrédients, additifs, peuvent être choisis parmi les fibres, les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires, les agents hydratants, les vitamines, les protéines, les céramides , les extraits végétaux , etc.. ; et leurs mélanges.

Bien entendu, l'homme du métier veillera à choisir ce ou ces éventuels composés complémentaires et/ou leur quantité, de manière telle que les propriétés des première et seconde compositions selon l'invention ne soient pas, ou substantiellement pas altérées par l'adjonction envisagée.

L'exemple qui suit illustre de manière non limitative l'invention.

**Exemple 1 : Kit de vernis à ongles**

a) Première composition ou "base":

| Nitrocellulose | 9,5 g |
|---|---|
| Tributyl citrate (Plastifiant) | 8 g |
| Résine alkyde | 13 g |
| Microsphères de verre (Prizmalite P2415BT) | 2 g |
| Pigments | 2,5 g |
| Agent épaississant | 1,3 g |
| Alcool Isopropylique | 7 g |
| Acétate d'éthyle | 25 g |
| Acétate de butyle | qsp 100 g |

Mode opératoire :

La nitrocellulose est solubilisée dans le mélange solvant (acétate d'éthyle et acétate de butyle), puis le plastifiant (tributyl citrate) et la résine alkyde sont ajoutés à l'ensemble.
L'agent épaississant et les pigments sont dispersés et broyés si nécessaire (par exemple dans un broyeur à sable) soit dans la totalité, soit dans une partie du mélange ci-dessus. Les microsphères de verre sont ensuite dispersées dans le mélange, puis l'ensemble est homogénéisé.

b) Seconde composition :

| Acétobutyrate de cellulose | 21 g |
|---|---|
| Tributyl citrate (Plastifiant) | 5,5g |
| Résine alkyde | 10 g |
| Résine acrylique | 2,5 g |
| Dimethicone | 0,2 g |
| Acétate d'éthyle | 15 g |
| Acétate de butyle | qsp 100 g |

L'acétobutyrate de cellulose est solubilisée dans le mélange solvant (acétate d'éthyle et acétate de butyle), puis le plastifiant (tributyl citrate), la résine alkyde puis la diméthicone sont ajoutés à l'ensemble.
On applique sur l'ongle une première couche de la première composition, puis, après séchage superficiel de cette première couche, on applique sur cette première couche une couche de la seconde composition puis on laisse sécher. Le film de vernis à ongles ainsi obtenu a été jugé d'aspect lisse, homogène, très brillant et présentant une bonne tenue et une bonne résistance à l'usure.

**Revendications**

1. Kit de maquillage ou de soin des ongles comprenant :

   i) une première composition comprenant, dans un premier milieu cosmétiquement acceptable un premier polymère filmogène, la première composition étant apte à former un film ayant une température de transition vitreuse Tg allant de 25 à 60°C,
   ii) une seconde composition comprenant un second milieu cosmétiquement acceptable, la seconde composition ayant un extrait sec supérieur ou égal à 15%.

2. Kit selon la revendication 2, **caractérisé en ce que** la première composition est apte à forme un film ayant une température de transition vitreuse Tg allant de 30 à 45°C.

**3.** Kit de maquillage ou de soin des ongles comprenant :

i) une première composition comprenant, dans un premier milieu cosmétiquement acceptable un polymère filmogène et des particules solides de forme sphérique et

ii) une seconde composition comprenant un second milieu cosmétiquement acceptable, ladite composition ayant un extrait sec supérieur ou égal à 15%.

**4.** Kit selon la revendication 1 ou 2, **caractérisé en ce que** la première composition comprend des particules solides de forme sphérique.

**5.** Kit selon l'une des revendications précédentes, **caractérisé en ce que** les particules solides de forme sphérique ont une taille moyenne inférieure ou égale à 50 μm, de préférence de 0,5 à 30 μm et mieux de 5 à 15 μm.

**6.** Kit selon l'une des revendications précédentes, **caractérisé en ce que** les particules solides de forme sphérique sont en un matériau choisi parmi le verre, les céramiques, les alumines, les silices, les silicates, le mica, le mica synthétique, le carbure de tungstène, la cellulose et ses dérivés, les résine de silicone, les cires naturelles ou synthétiques, les métaux, les oxydes métalliques, les alliages, les matériaux d'origine végétale d'origine minérale ou les matériaux issus de sécrétion animale et leurs mélanges.

**7.** Kit selon l'une des revendications précédentes, **caractérisé en ce que** les particules solides de forme sphérique sont des particules de verre.

**8.** Kit selon l'une des revendications précédentes, **caractérisé en ce que** les particules solides de forme sphérique représentent de 0,1 à 30% en poids par rapport au poids total de la composition, de préférence de 0,5 à 10 % et mieux de 1 à 5 % en poids.

**9.** Kit selon l'une des revendications précédentes, **caractérisé en ce que** la première composition est apte à former un film ayant un pouvoir amortissant tangente delta supérieur à 0,7.

**10.** Kit selon l'une des revendications précédentes, **caractérisé en ce que** la première composition est apte à former un film ayant une dureté Persoz allant de 30 à 60 secondes.

**11.** Kit selon l'une des revendications précédentes, **caractérisé en ce que** la première composition a une viscosité allant de 0,15 à 0,5 Pa.s.

**12.** Kit selon l'une des revendications précédentes, **caractérisé en ce que** le premier milieu cosmétiquement acceptable de la première composition comprend un milieu choisi parmi un milieu solvant organique comprenant au moins un solvant organique, un milieu aqueux et leur mélange.

**13.** Kit selon la revendication précédente, **caractérisé en ce que** le milieu solvant organique, le milieu aqueux ou la somme de ces deux milieux est présent en une teneur totale comprise entre 1 et 90 % en poids, notamment allant de 5 à 60 % en poids, et en particulier allant de 15 à 40 % en poids, par rapport au poids total de la première composition.

**14.** Kit selon l'une des revendications précédentes, **caractérisé en ce que** le premier polymère filmogène est choisi dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

**15.** Kit selon l'une des revendications précédentes, **caractérisé en ce que** le premier polymère filmogène représente de 1 à 70% en poids par rapport au poids total de la composition et mieux de 10 à 45% en poids.

**16.** Kit selon l'une des revendications précédentes, **caractérisé en ce que** la seconde composition a un extrait sec de 20%, de préférence de 25%.

**17.** Kit selon l'une des revendications précédentes, **caractérisé en ce que** la seconde composition a une viscosité allant de 0,15 à 1,5 Pa.s.

**18.** Kit selon l'une des revendications précédentes, **caractérisée en ce que** le milieu cosmétiquement acceptable de la seconde composition comprend un milieu choisi parmi un milieu solvant organique comprenant au moins un solvant organique, un milieu aqueux et leur mélange.

**19.** Kit selon la revendication précédente, **caractérisé en ce que** le milieu solvant organique, le milieu aqueux ou la somme de ces deux milieux est présent en une teneur totale comprise entre 1 et 90 % en poids, notamment allant de 5 à 60 % en poids, et en particulier allant de 15 à 40 % en poids, par rapport au poids total de la seconde composition.

**20.** Kit selon l'une des revendications précédentes, **caractérisé en ce que** la seconde composition comprend un second polymère filmogène.

**21.** Kit selon la revendication précédente, **caractérisé en ce que** le second polymère filmogène est choisi dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

**22.** Kit selon l'une des revendications précédentes, **caractérisée en ce que** le second polymère filmogène est présent en une teneur en matières sèches supérieure ou égale à 5%, de préférence supérieure à 10%, mieux supérieure à 15%, et encore mieux supérieure à 20%.

**23.** Kit selon l'une des revendications précédentes, **caractérisé en ce que** la première et/ou la seconde composition comprend un plastifiant.

**24.** Kit selon la revendication précédente, **caractérisé en ce que** le plastifiant représente de 0,1% à 15% en poids, par rapport au poids total de chacune des première ou seconde compositions, et de préférence de 0,5% à 10% en poids.

**25.** Kit selon l'une des revendications précédentes, **caractérisé en ce que** la première et/ou la seconde composition comprend une matière colorante.

**26.** Kit selon la revendication précédente, **caractérisée en ce que** la matière colorante est présente en une teneur allant de 0,01% à 25% en poids, et de préférence de 2 à 7 % en poids par rapport au poids total de chacune des première ou seconde compositions.

**27.** Kit selon l'une des revendications précédentes, **caractérisé** en que la première et/ou la seconde composition comprend un additif choisi parmi les fibres, les agents de coalescence, les agents épaississants, les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires, les agents hydratants, les vitamines, les protéines, les céramides, les extraits végétaux et leurs mélanges.

**28.** Procédé cosmétique de maquillage ou soin non thérapeutique des ongles consistant à appliquer sur les ongles une première couche d'une première composition comprenant un premier milieu cosmétiquement acceptable et un premier polymère filmogène, la première composition étant apte à former un film ayant une température de transition vitreuse Tg allant de 25 à 60°C, puis à appliquer sur tout ou partie de la première couche et avant séchage de la première couche, une seconde couche d'une seconde composition comprenant un second milieu cosmétiquement acceptable, la seconde composition ayant un extrait sec supérieur ou égal à 15%.

**29.** Procédé cosmétique de maquillage ou soin non thérapeutique des ongles consistant à appliquer sur les ongles une première couche d'une première composition comprenant un premier milieu cosmétiquement acceptable et un premier polymère filmogène, la première composition étant apte à former un film ayant une température de transition vitreuse Tg allant de 25 à 60°C, puis, après séchage total ou partiel de la première couche, à appliquer sur tout ou partie de la première couche une seconde couche d'une seconde composition comprenant un second milieu cosmétiquement acceptable, la seconde composition ayant un extrait sec supérieur ou égal à 15%.

**30.** Procédé cosmétique de maquillage ou soin non thérapeutique des ongles consistant à appliquer sur les ongles une première couche d'une première composition comprenant un premier milieu cosmétiquement acceptable, un premier polymère filmogène et des particules solides de forme sphérique, puis à appliquer sur tout ou partie de la première couche, et avant séchage de la première couche, une seconde couche d'une seconde composition comprenant un second milieu cosmétiquement acceptable, la seconde composition ayant un extrait sec supérieur ou égal à 15%.

31. Procédé cosmétique de maquillage ou soin non thérapeutique des ongles consistant à appliquer sur les ongles une première couche d'une première composition comprenant un premier milieu cosmétiquement acceptable, un premier polymère filmogène et des particules solides de forme sphérique, puis, après séchage total ou partiel de la première couche, à appliquer sur tout ou partie de la première couche une seconde couche d'une seconde composition comprenant un second milieu cosmétiquement acceptable, la seconde composition ayant un extrait sec supérieur ou égal à 15%.

32. Utilisation d'un kit selon quelconque des revendications 1 à 27, pour obtenir un film déposé sur l'ongle, présentant des propriétés de tenue et/ou une résistance à l'usure améliorée.

**Office européen des brevets**

## RAPPORT DE RECHERCHE EUROPEENNE

Numéro de la demande

EP 04 29 1227

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 01/82865 A (REVLON CONSUMER PROD CORP) 8 novembre 2001 (2001-11-08) * page 9, ligne 25 - page 10, ligne 35; revendications 17-20 * | 1-32 | A61K7/043 |
| X | WO 99/56705 A (PROCTER & GAMBLE) 11 novembre 1999 (1999-11-11) * page 9, ligne 27 - page 10, ligne 10; revendications 10-15; exemples 1-3 * | 1-32 | |
| X | WO 97/42930 A (REVLON CONSUMER PROD CORP) 20 novembre 1997 (1997-11-20) * page 8, ligne 2-5; revendications 28,29 * | 1-32 | |
| A | WO 02/05762 A (OREAL ;BERNARD PASCALE (FR); HADASCH ANKE (FR); TOUMI BEATRICE (FR) 24 janvier 2002 (2002-01-24) * page 7, ligne 21-31; revendications 1-6 * | 1-32 | |
| | | | **DOMAINES TECHNIQUES RECHERCHES (Int.Cl.7)** |
| | | | A61K |

Le présent rapport a été établi pour toutes les revendications

| Lieu de la recherche | Date d'achèvement de la recherche | Examinateur |
|---|---|---|
| Munich | 13 septembre 2004 | Lindner, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**  EP 04 29 1227

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-09-2004

| Document brevet cité au rapport de recherche | | Date de publication | Membre(s) de la famille de brevet(s) | | Date de publication |
|---|---|---|---|---|---|
| WO 0182865 | A | 08-11-2001 | AU | 5928001 A | 12-11-2001 |
| | | | CA | 2407205 A1 | 08-11-2001 |
| | | | EP | 1278501 A2 | 29-01-2003 |
| | | | WO | 0182865 A2 | 08-11-2001 |
| | | | US | 2002018759 A1 | 14-02-2002 |
| | | | ZA | 200208935 A | 09-12-2003 |
| WO 9956705 | A | 11-11-1999 | US | 6306375 B1 | 23-10-2001 |
| | | | AU | 3876199 A | 23-11-1999 |
| | | | CN | 1299267 T | 13-06-2001 |
| | | | EP | 1083862 A2 | 21-03-2001 |
| | | | JP | 2002513735 T | 14-05-2002 |
| | | | WO | 9956705 A2 | 11-11-1999 |
| WO 9742930 | A | 20-11-1997 | US | 5772988 A | 30-06-1998 |
| | | | AU | 707799 B2 | 22-07-1999 |
| | | | AU | 3062097 A | 05-12-1997 |
| | | | CA | 2226567 A1 | 20-11-1997 |
| | | | EP | 0845973 A1 | 10-06-1998 |
| | | | JP | 11509869 T | 31-08-1999 |
| | | | WO | 9742930 A1 | 20-11-1997 |
| | | | ZA | 9704081 A | 09-12-1997 |
| WO 0205762 | A | 24-01-2002 | FR | 2811546 A1 | 18-01-2002 |
| | | | EP | 1303248 A1 | 23-04-2003 |
| | | | WO | 0205762 A1 | 24-01-2002 |
| | | | JP | 2004503574 T | 05-02-2004 |
| | | | US | 2004052745 A1 | 18-03-2004 |

EPO FORM P0460

Pour tout renseignement concernant cette annexe : voir Journal Officiel de l'Office européen des brevets, No.12/82